# EUROPEAN PATENT APPLICATION

(11) **EP 1 145 645 A1**
(43) Date of publication of application: **17.10.2001**
(21) Application number: 00108350.0
(22) Date of filing: 14.04.2000
(51) Int. Cl.: A23K 1/00, G01N 33/02, G01N 21/35

(54) **Production of animal feed**

(71) Applicant: AVENTIS ANIMAL NUTRITION S.A., 92164 Antony Cedex (FR)
(72) Inventor: Haeffner, Jürgen, 55218 Ingelheim am Rhein (DE); Harari Guy, Dunwoody GA 30338 (US); D'Alfonso, Tom, 92330 Sceaux (FR)
(74) Representative: Hoey, Shona

(57) **Abstract**

A method of selecting and enhancing of feedstuffs for use in an animal feed product by analysing the statistical distribution of the nutritional content of batches of the feedstuff and calculating the number of batches within a predetermined range of a desirable nutrient composition. The cost of enhancing the feedstuff by adding nutrient additives is calculated and batches of the feedstuff are accepted or rejected, with the enhancement step only applied to the accepted batches. The method can be applied to products such as soyabean meal and corn which are major constituents of animal feed. It produces a feedstuff with a lower than natural variance in nutrient composition and a guarantee of a desired nutritional composition without the need for systematic overformulation of the product.

## Description

The present invention relates to the production of animal feed and in particular to the selection and enhancement of raw materials for use in animal feed production.

Animal feeds typically consist of a mixture of materials. For instance, a typical composition for a feed for poultry is 25% soyabean meal, 50% corn, 20% byproducts suitable for animal feed and 5% minerals, vitamins, supplements and other feed additives. Feeds for other animals have different compositions, and soyabean meal is one of the most important vegetable protein sources for animal feeds in general. For instance, it is one of the main components of feed for poultry.

In order to achieve the most efficient growth of animals, the diet needs to be carefully controlled and thus the nutrient composition of the feedstuff is of high importance. However, natural raw materials have a high variation in nutrient composition. For instance, soyabean meal is generally classified into high and low protein products, namely high protein soyabean meal (HPSBM) with 49% protein and low protein soyabean meal (LPSBM) with 44% protein. High protein soyabean meal is of a higher price than low protein soyabean meal but in reality neither have a fixed level of protein, but vary within certain tolerance limits about the average value. In addition, soyabean meal also provides protein to the diet and the comprising amino acids and other nutients. These other nutrients are very important in obtaining optimal performance of the feed but the amount of the amino acids, respectively the first limiting amino acids: lysine, methionine, threonine and tryptophane, vary widely between different batches of soyabean meal, for example the coefficient of variation (CV) of lysine and methionine composition amongst batches of soyabean meal can be approximately 10%.

In order to guarantee the level of desired nutrients in the feed it has been proposed to measure the level of nutrient in raw material for the feed, and to supplement that level where necessary with additional components, for instance, synthetic, nutrients. For instance, synthetic methionine can be added to the soyabean meal. In the case of the amino acid levels in soyabean meal, these can be assessed by inspecting the near infrared spectrum of the soyabean meal. It has been found that the near infrared spectrum (NIRS) of soyabean meal is dependent upon the amino acid content. By establishing a database relating the NIR spectrum to the amino acid levels (measured by other means), it is possible to use the NIR spectrum of a given batch of soyabean meal to assess its amino acid content.

Such techniques are described in «Near-Infrared Reflectance Spectroscopy in Precision Feed Formulation» by Van Kempen and Simmins; Applied Poultry Science, 1997, pp471-475 and «NIRS may provide rapid evaluation of amino acids» by Van Kempen and Jackson, Feedstuffs, December 2, 1996.

The known method may be applied to any of a number of feedstuffs and their comprising nutrients. For example, the caloric content, and specifically the metabolizable energy content of corn or the fat composition of bakery by-product meal are examples to which the method applies.

A more typical way of providing a guaranteed level of nutrient is to assess the natural variation of the level of nutrient in the raw material and to add a sufficient amount of supplement to all batches of the raw material to achieve a guaranteed level. Clearly this techniques does not reduce the natural variation in level, but raises the average level to the desired level. However, if the natural content of nutrient is overestimated, the requirements of the animals are not met and the performance is reduced. To minimise this risk safety margins are used for the formulation of the feed. These safety margins result in a systematic overformulation of specific nutrient ingredients and such overformulation is costly and reduces efficiency.

The present invention provides a method of analysing, selecting and enhancing raw materials for use in animal feed products in a manner which eliminates the systematic overformulation, while guaranteeing a desired level of nutrient in the supplemented product.

In more detail, according to one aspect, the present invention provides a method comprising the steps of:
analysing the nutritional composition of batches of a raw material for use in an animal feed product;
comparing the nutritional composition with a predetermined nutritional composition;
calculating the amount of supplemental nutrient needed to bring the composition of the batch to the predetermined nutritional composition;
screening the batches to reject those for which the amount of supplemental nutrient needed is greater than a threshold value and to accept those for which the amount of supplemental nutrient needed is less than a threshold value; and
supplementing only the accepted batches of raw material with the calculated amount of supplemental nutrient.

Preferably the method further includes a step of analysing the statistical distribution (i.e. frequency) of the nutritional content of batches of the raw material, assessing the number of batches for which the amount of supplemental nutrient needed is less than the threshold value, and performing the screening and supplemental step on condition that the number of batches is greater than the predetermined value. This may conveniently be done by setting a nutritional composition threshold and comparing the nutritional content of each batch with the threshold, and rejecting those falling below the threshold.

The nutritional composition threshold may define the boundary of a range of nutritional compositions near to a predefined nutritional composition profile and the step of analysing the statistical distribution of the nutritional content of the batches of the raw material comprises estimating the percentage of batches that are clustered within that range.

In the method of the present invention, the nutritional composition can be the amount of at least one amino acid such as methionine or lysine in the raw material. This can be measured by a fast, convenient, «in vitro» analysis technique such as near infrared reflectance spectroscopy (NIRS). In this case a database can be established relating the NIRS spectrum of the raw material to the amino acid content, and subjecting incoming batches to spectroscopy, then using the database to derive the amino acid content.

In the method of the present invention, the digestibility of the nutrient in the raw material can also be measured. This is measured in the same way for the amount of amino acid, namely analysis using NIRS. Again, a data base is established as above. For the purposes of the present invention, digestibility is defined as the proportion of a particular nutrient that is actually available to the animal to metabolise, for example "available phosphorus" refers to the amount of phosphorous that is metabolised by the animal; digestible amino acids are the amount of amino acids metabolised by the animal and metabolisible energy is the amount of calories in the feed that is actually metabolised by the animal.

Alternatively the nutritional composition can be the amount of fat or oil in the raw material.

The method is particularly suitable for application to raw materials such as soyabean meal and corn which are major components in animal feed.

The invention is preferably performed by an analysis and enhancement system which includes an analysis device (which may be an NIRS device), a data processing device such as a programmed computer which executes the comparison and calculation steps, and a nutrient supplement supply device for supplementing the accepted batches of raw material. Thus the invention encompasses a computer program adapted to control and execute the method on such a system.

The result of applying the invention is the production of an enhanced raw material with a consistent, desired level of nutritional value, and with a lower than natural variance in the nutritional value.

The invention will be further described by way of example with reference to the accompanying drawings in which:-
Figure 1 is a flow diagram showing the overall decision making flow in an embodiment of the invention;
Figure 2 is a graph illustrating the relationship between digestible lysine content and protein content in maize;
Figure 3 is a schematic diagram showing the invention as applied to the processing of soyabean meal;
Figure 4 illustrates an NIR spectrum of methionine;
Figure 5 illustrates an NIR spectrum of soyabean meal; and
Figure 6 illustrates the digestible methionine content of natural soyabean meal and an animal feed product containing the enhanced soyabean meal.

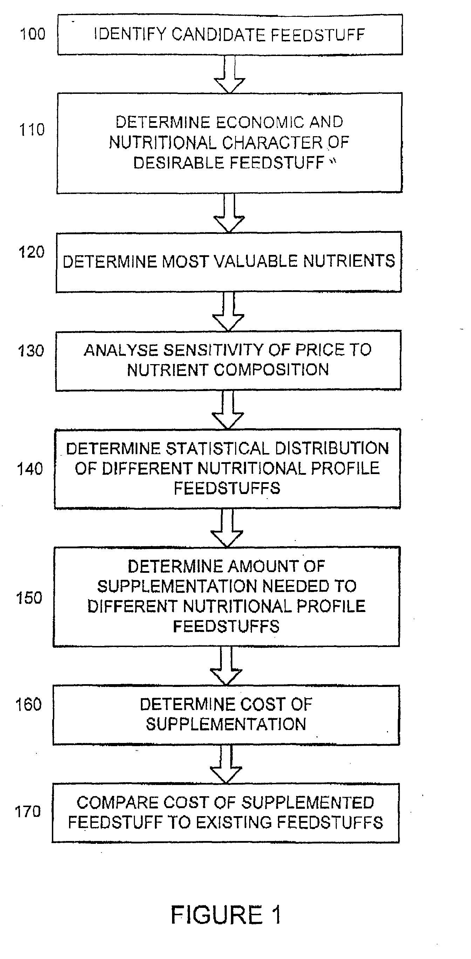

Figure 1 illustrates the overall decision flow of an embodiment of the invention and this will be described below while making reference to a particular application of the invention to the processing of soyabean mean which, as discussed above, is one of the major constituents of animal feed.

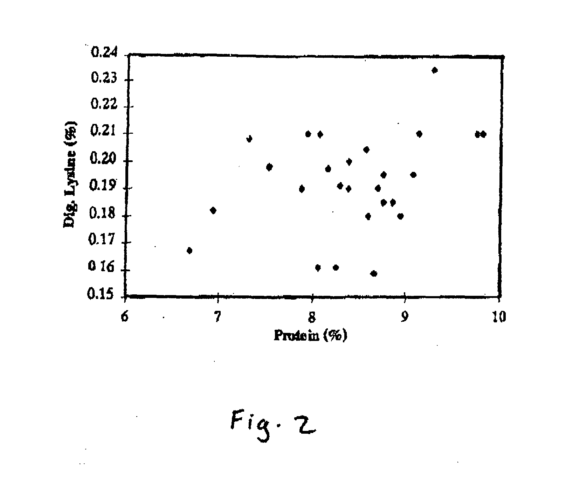

Referring to Figure 1, in general terms the first step in the process is to identify a candidate feedstuff. Typically examples are soyabean meal or corn. These feedstuffs can provide protein and other nutrients, such as amino acids, to the animal. However, the feedstuffs do not have a consistent content. Figure 2 illustrates in the case of corn the variability in digestible lysine content as a function of the protein content of randomly selected batches. It can be seen that even if one were to select a consistent protein content, the digestible lysine content would vary markedly.

Therefore with the invention the next step 110 is to determine the nutritional characteristics and economic characteristics (primarily acceptable price) of a desirable or ideal feedstuff. In the case of soyabean meal being used for poultry feed, as mentioned above, the soyabean meal is available basically as a high protein product (49%) or a low protein product (44%). The high protein product is more expensive at $160 per ton than the low protein product at $150 per ton. While the high protein product may be expected to have high levels of amino acid, in fact it is found that there is a high variability in the amino acid level in both types of soyabean meal. Thus even use of the high protein product cannot guarantee that the final feedstuff is of optimal value to the animal. Further, there is a difficult balance to be struck between the use of lower quantities of the more expensive high protein meal, or higher quantities of the lower protein cheaper soyabean meal.

According to the invention therefore in step 120 the most valuable nutrients in the feedstuff are identified. In the case of soyabean meal these are methionine and lysine. It is important to understand not all of the nutrient present may be digestible to the animal. Thus the total composition and the digestibility of the nutrients need to be considered. In this example in the case of soyabean meal it is desirable to have a digestibility of approximately 90%, as determined by NIRS screening.

Then in step 130 the sensitivity of the feedstuff price to the nutrient composition is analysed. In the case of soyabean meal it was desirable to have the methionine and lysine content identical, if possible, with high protein soyabean meal.

It is then necessary to consider in step 140 whether there is sufficient feedstuff available which can be economically enhanced with supplemental nutrient to satisfy the desired nutritional profile and thus a statistical analysis of the feedstuff is made. In the case of soyabean meal it is found that the nutrient composition, particularly in terms of amino acid content is not uniformly distributed. Instead the batches cluster in terms of their amino acid content. With the invention clusters are identified of batches of low protein soyabean meal which, despite being low in protein, are high in both total and digestible composition of amino acid. These batches require only a small amount of supplementation to achieve the desired nutritional profile, namely an amino acid profile identical to that of high protein soyabean meal.

With regard to the statistical distribution of the feedstuff it is then necessary to calculate, in step 150 the amount of supplementation needed for each of the batches in the selected clusters. This can be done by using a Monte Carlo simulation to determine the amount of supplementation needed on average and the distribution of that supplementation (minimum and maximum supplementation needed). In the case of soyabean meal the clusters of low protein soya bean meal which are close to the high protein soyabean meal in terms of amino acid content, are selected such that the minimum supplementation of methionine and lysine needed is 0% and the maximum 0.1%. In step 160 the cost of such supplementation, in terms of the raw material cost (for LPSBM this is $150 per ton), the cost of the supplement and the cost of the monitoring, analysis and supplementing equipment, is calculated.

Then in step 170 the cost of producing the supplemented feedstuff is compared to the cost of existing competing feedstuff, such as HPSBM. Table 1 below illustrates the nutrient composition and economic value of soyabean meal in the case of LPSBM, HPSBM and the enhanced product, marked as guaranteed soyabean meal (GSBM). It can be seen that the price of LPSBM is $150 per ton, the price of HPSBM is $160 per ton and the price of GSBM at a selling price of$157.50 per ton still results in a reduced feed cost compared to the use of HPSBM. This is despite the fact that the cost of the additive needed is $1.60 per ton and the estimated cost of the equipment for screening the product and adding the additive is $0.75 per ton. It is therefore demonstrated that using the procedure illustrated in Figure 1 it is possible to select and enhance a raw material for a feedstuff in a manner which improves the nutritional value, while maintaining economic advantage for the user of the feedstuff. For example, batches of corn may be analyzed for caloric content. The amount of calories in corn that is digestible and metabolized by the animal is commonly referred to as "metabolizable energy" (ME). Following the invention described in Figure 1.
1. Corn is identified as the feedstuff.
2. Clusters of corn batches with high ME and low cost can be discovered
3. Sufficient batches of high ME corn exist for the market.
4. Supplemented energy in the form of vegetable oil may be used.
5. The net economic value of this new feedstuff can be calculated

**Table 1**

| Nutrient composition and economic value of soyabean meal with guaranteed composition | | | | |
|---|---|---|---|---|
| | LPSBM | HPSBM | GSBM | COMMENTS |
| Protein | 44% | 49% | 44% | same as Soy 44% |
| Lysine | 2,74% | 3,07% | 3,07% | same as Soy 49% |
| Methionine | 0,60% | 0,68% | 0,68% | same as Soy 49% |
| TSAA | 1,23% | 1,39% | 1,35% | |
| Threonine | 1,72% | 1,94% | 1,83% | |
| Metabolizable Energy | 2244 | 2420 | 2244 | same as Soy 44% |
| Other Nutrients | X | Y | X | same as Soy 44% |
| Meth 99 | 0% | 0% | 0,08% | Supplement needed |
| Raw Material Cost | 150,00 | 160,00 | 151,63 | |
| Value Net Value Added | 150,00 | 160,00 | 157,50 $5.87 | selling price per ton |
| Feed Price Difference | 145,75 | 145,50 | 145,00 -0,50 | reduced feed costs per ton |
| Nutrient Variance | 4.56% | 4,25% | 3.40% | reduced nutrient variation |
| Difference | | | -20% | |

It is also important to note that the variance in nutrient content is significantly reduced, even compared to the HPSBM. The variance in nutrient content of LPSBM is 4.56%, in the case of HPSBM it is 4.25%, but in the case of GSBM it is only 3.4%. Thus the feedstuff manufacturer can use the enhanced produce without the need for monitoring the content or systematic overformulation, while still being confident of providing a feedstuff of guaranteed value to the animal.

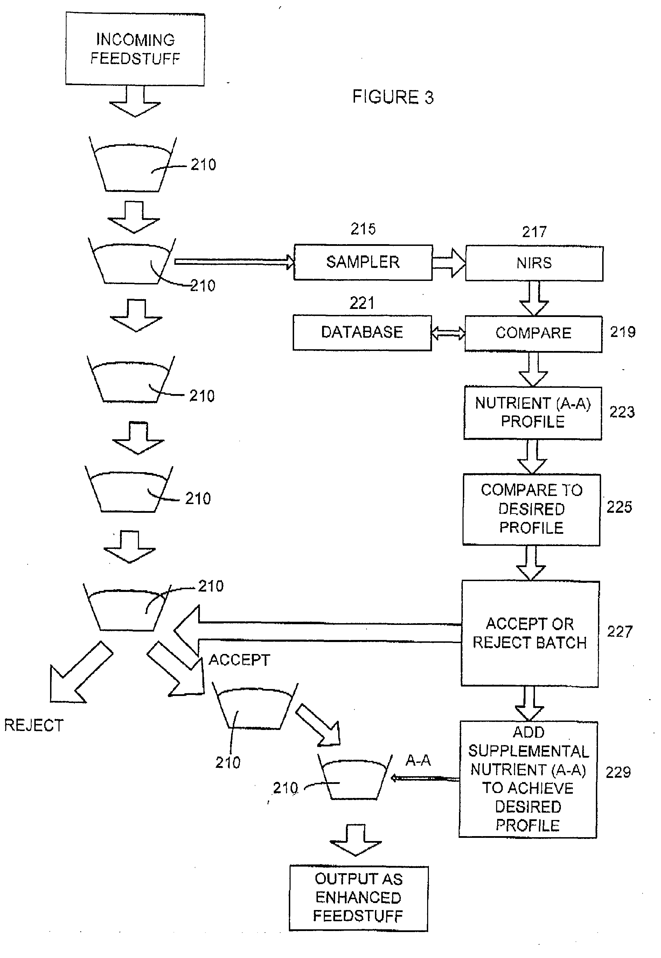

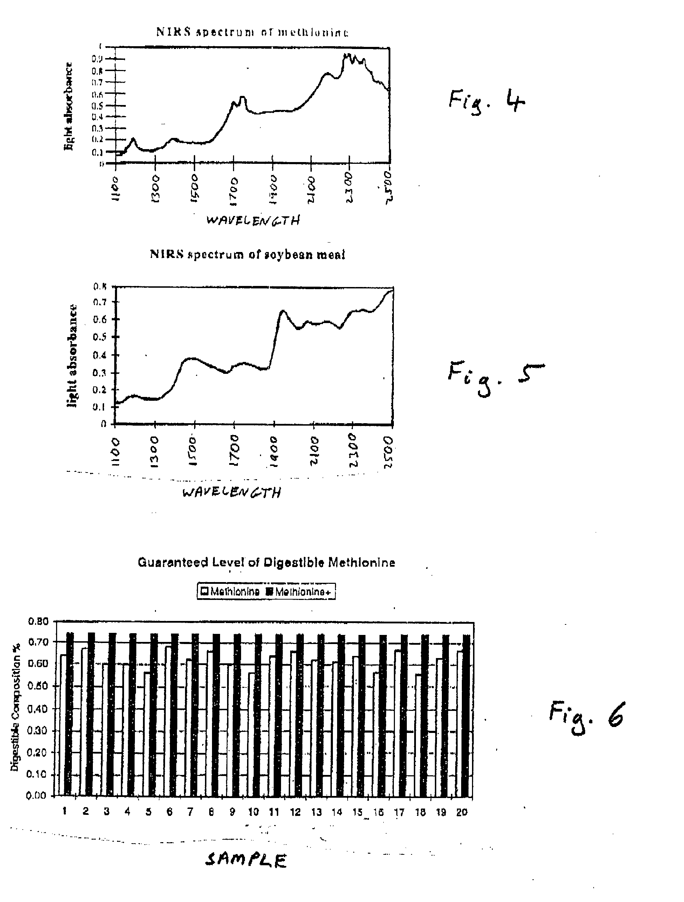

Figure 3 illustrates schematically the application of the system in the production of an enhanced soyabean meal. Batches of soyabean meal 210 are sampled by a sampler 215 and subjected to near infrared reflectance spectroscopy by spectrometer 217. The spectrum is compared at 219 to spectra stored in a database 221 relating the spectra to the amino acid content. Figure 4 illustrates the NIRS spectrum of methionine and Figure 5 illustrates the NIRS spectrum of soyabean meal. By comparing Figures 4 and 5 it will be seen that a direct quantification of the soyabean meal composition is not possible because the spectrum of soyabean meal lacks distinguishable peaks and the spectrum of Figure 5 is actually a composite spectrum of different substances present in the soyabean meal. Therefore the preferred way of assessing the amino acid content from the spectrum is to establish the database 221 relating spectra to amino acid content measured by other analysis techniques. The result of the comparison at 219 is therefore a nutrient (amino acid) profile 223. This is compared at 225 to the desired profile established by considering the ideal diet for the animal. The batch is then accepted or rejected at step 227 and, if accepted, a nutrient, in this case amino acid additive is added at step 229 to achieve the desired nutrient profile in the feedstuff.

Figure 6 illustrates the level of digestible methionine in the incoming soyabean meal, which can be seen to vary markedly, and the level in the complete feed, i.e. after enhancement by the addition of synthetic methionine. It can be seen that a guaranteed level of 0.74% can be achieved, compared to the 0.62% average in the raw product.

In summary, therefore the embodiment described allows the selecting and enhancing of feedstuffs for use in an animal feed product by analysing the statistical distribution of the nutritional content of batches of the feedstuff and calculating the number of batches within a predetermined range of a desirable nutrient composition. The cost of enhancing the feedstuff by adding nutrient additives is calculated and batches of the feedstuff are accepted or rejected, with the enhancement step only applied to the accepted batches.

It will be understood that the product of this process is of value in that it has a desired nutritional profile, with lower than natural variance, while being available in sufficient amounts and value which is acceptable to feedstuff users. In the particular example of soyabean meal explained above, there is additional advantage in that the product has a low protein content, yet high amino acid content. The low protein content is effective in reducing the environmental pollution produced by animals consuming the final feed product. The invention makes it possible to produce such a product by selecting the raw material with reference to the nutritional content so avoiding systematic overformulation. Further, statistical analysis of the frequency distribution of the nutritional content amongst batches of the feedstuff allows the optimisation of the selection of the raw material having regard to the amount of product to be produced, its cost of production, and the desired nutritional profile.

## Claims

1. A method comprising the steps of:
analysing the nutritional composition of batches of a raw material for use in an animal feed product;
comparing the nutritional composition with a predetermined nutritional composition;
calculating the amount of supplemental nutrient needed to bring the composition of the batch to the predetermined nutritional composition;
screening the batches to reject those for which the amount of supplemental nutrient needed is greater than a threshold value and to accept those for which the amount of supplemental nutrient needed is less than a threshold value; and
supplementing only the accepted batches of raw material with the calculated amount of supplemental nutrient.

2. A method according to claim 1 further comprising the step of analysing the statistical distribution of the nutritional content of batches of the raw material; assessing on the basis of the statistical distribution the number of batches for which the amount of supplemental nutrient needed is less than a threshold value; and on condition that said number of batches is greater than a predetermined value performing said screening and supplementing steps.

3. A method according to claim 2 further comprising setting with respect to said threshold value and said statistical distribution a nutritional composition threshold and wherein the step of screening comprises comparing the nutritional content of each batch with said nutritional composition threshold and rejecting those batches falling below said nutritional composition threshold.

4. A method according to claim 3 wherein the nutritional composition threshold defines a boundary of a range of nutritional compositions compared to a predefined nutritional composition profile and said step of analysing the statistical distribution of the nutritional content of batches of the raw material comprises estimating the percentage of batches that are clustered within said range.

5. A method according to claim 1, 2, 3 or 4 wherein the step of analysing the nutritional composition comprises measuring the amount of at least one amino acid in the raw material.

6. A method according to claim 1, 2, 3 or 4 wherein the step of analysing the nutritional composition comprises measuring the digestibility of the nutrient in the raw material.

7. A method according to claim 5 wherein said amino acid is at least one of methionine and lysine.

8. A method according to any one of the preceding claims wherein the step of analysing the nutritional composition comprises measuring the amount of at least one amino acid in the raw material by near infrared reflectance spectroscopy (NIRS).

9. A method according to any one of the preceding claims wherein the step of analysing the nutritional composition comprises measuring the amount of fat in the raw material.

10. A method according to any one of the preceding claims wherein the raw material is soyabean meal.

11. A method according to any one of claims 1 to 10 wherein the raw material is corn.

12. A computer program comprising program code means for controlling and executing the method of any one of the preceding claims.

13. A computer readable storage medium encoding the computer program of claim 12.

14. An analysis and enhancement system for analysing and enhancing the nutritional composition of raw material for use in the production of animal feed, the system comprising: an analysis device for analysing the nutritional composition of batches of the raw material; a data processing device for comparing the nutritional composition with a predetermined nutritional composition, calculating the amount of supplemental nutrient needed to bring the composition of the batch to the predetermined nutritional composition, screening the batches to reject those for which the amount of supplemental nutrient needed is greater than a threshold value and to accept those for which the amount of supplemental nutrient needed is less than a threshold value; and a nutrient supplement supply device for supplementing only the accepted batches of raw material with the calculated amount of supplemental nutrient.

15. An analysis and enhancement system according to claim 14 wherein said analysis device comprises a near infrared reflectance spectrometer for performing near infrared reflectance spectroscopy (NIRS) on said batches to obtain an NIR spectrum of the raw material, and a database storing information relating the NIR spectrum of the raw material to the nutritional content thereof.

16. An analysis and enhancement system according to claim 14 or 15 wherein said database stores information relating the NIR spectrum of the raw material to the amino acid content thereof.

17. A nutritionally supplemented raw material for use in the manufacture of an animal feed product and produced by the method of any one of claims 1 to 10.
